# EUROPEAN PATENT APPLICATION

(11) **EP 2 263 675 A2**
(43) Date of publication of application: **22.12.2010**
(21) Application number: 10010923.0
(22) Date of filing: 09.06.2006
(51) Int. Cl.: A61K 31/58, A61K 9/20, A61K 9/16, A61K 9/50, A61P 25/22

(54) **Pharmaceutical compositions of a neuroactive steroid and uses thereof**

(30) Priority: 09.06.2005 US 688905 P
(62) Divisional of application: 10000305.2
(71) Applicant: EURO-CELTIQUE S.A., 1653 Luxembourg (LU)
(72) Inventor: Woodward, Richard M., Phoenixville, PA 19460-4638 (US)
(74) Representative: Maiwald Patentanwalts GmbH

(57) **Abstract**

The present invention relates to a pharmaceutical composition, comprising 3α-hydroxy-3β-methoxymethyl-21-(1'-imidazolyl)-5a-pregnan-20-one or a pharmaceutically-acceptable salt or solvate thereof, and one or more pharmaceutically-acceptable excipients, wherein the composition provides steady state plasma levels of 3α-hydroxy-3β-methoxymethyl-21-(1'-imidazolyl)-5α-pregnan-20-one in a range of from 5 ng/mL to 500 ng/mL for a duration of from 6 h to 24 h following administration.

## Description

### Background of the invention

### Field of the invention

The present invention relates to the field of medicinal chemistry and to pharmaceutical compositions useful for treating mood disorders and the like. More specifically, the invention relates to pharmaceutical compositions of 3α-hydroxy-3β-methoxymethyl-21-(1'-imidazolyl)-5α-pregnan-20-one or a pharmaceutically-acceptable salt or solvate thereof, that provide sustained therapeutic plasma levels of 3α-hydroxy-3β-methoxymethyl-21-(1'-imidazolyl)-5α-pregnan-20-one. The present invention also relates to methods of treating these disorders by administering the pharmaceutical compositions.

### Related background art

3α-Hydroxy-3β-methoxymethyl-21-(1'-imidazolyl)-5α-pregnan-20-one is a synthetic neuroactive steroid. Its primary molecular target is the γ-aminobutyric acid type A (GABA_{A}) receptor, where it acts as a positive allosteric modulator of channel function. Like other classes of GABA_{A} modulators, such as benzodiazepines and other benzodiazepine site ligands, neuroactive steroids have a number of potential indications, such as for the treatment of sleep disorders (*see, e.g.,* Edgar, D.M., et al., J. Pharmacol. Exp. Ther. 282:420-29 (1997), Friess, E., et al., Am. J. Physiol. 272 (Endocrin. Metab. 35):E885-91(1997)), anxiety (*see, e.g.,* Purdy, R.H., et al., Proc. Natl. Acad. Sci. 88:4553-57 (1991), Vanover, K.E., et al., J. Pharmacol. Exp. Ther. 295:337-45 (2000), Ströhle, A., et al., Arch. Gen. Psychiatry 60:161-68 (2003)), depression (*see, e.g.,* Dong, E., et al., Proc. Natl. Acad. Sci. 98:2849-54 (2001), Rupprecht, R. and Holsboer, F., Trends Neurosci. 22:410-16 (1999), Uzunova, V., et al., Proc. Natl. Acad Sci. 95:3239-44 (1998)), epilepsy (*see, e.g.,* Carter, R.B., et al., J. Pharmacol. Exp. Ther. 280:1284-95 (1997), Laxer, K., et al., Epilepsia 41:1187-94 (2000), Kerrigan, J.F., et al., Epilepsy Res. 42:133-39 (2000)), and premenstrual syndrome (PMS) and premenstrual dysphoric disorder (PMDD) (*see, e.g.,* Rapkin, A.J., et al., Obs. Gyn. 90:709-14 (1997), Monteleone, P., et al., Eu. J. Endocrinol. 142:269-73 (2000), Smith, M.J., et al., Biol. Psychiatry 54:757-62 (2003)).

U.S. Patent Nos. 5,939,545 and 6,277,838 discuss compounds of the formula: 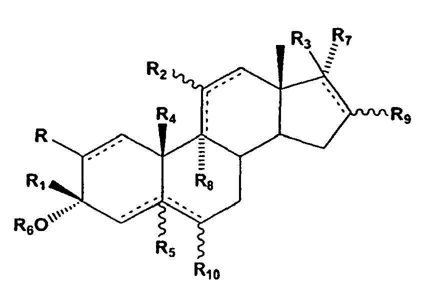 as useful in the treatment or prevention of stress or anxiety, mood disorders including depression, premenstrual syndrome or postnatal depression. *See, e.g.,* '838, col. 60, line 60 through col. 61, line 65. One such compound is 3α-hydroxy-3β-methoxymethyl-21-(1'-imidazolyl)-5α-pregnan-20-one: 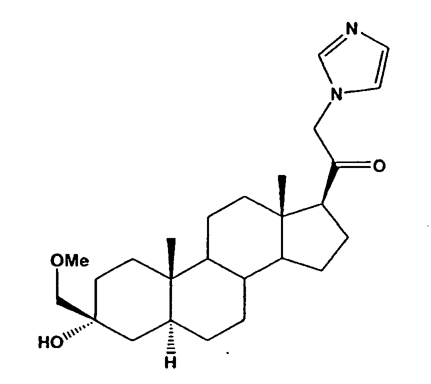 *See* Vanover, K.E., et al., Psychopharmacology 155:285-91 (2001).

### Summary of the invention

A first aspect of the present invention is directed to a pharmaceutical composition comprising 3α-hydroxy-3β-methoxymethyl-21-(1'-imidazolyl)-5α-pregnan-20-one or a pharmaceutically-acceptable salt or solvate thereof, and one or more pharmaceutically-acceptable excipients, that provides steady state therapeutic plasma levels of 3α-hydroxy-3β-methoxymethyl-21-(1'-imidazolyl)-5α-pregnan-20-one. Target plasma levels for treating mood disorders and the like in humans range from about 5 ng/mL to about 500 ng/mL, particularly from about 50 ng/mL to about 250 ng/mL. CNS and other side effects are expected to occur at plasma levels greater than about 500 ng/mL.

A further aspect of the present invention is directed to a method of treating a condition or disorder which treatment benefits from sustained therapeutic plasma levels of 3α-hydroxy-3β-methoxymethyl-21-(1'-imidazolyl)-5α-pregnan-20-one. In one embodiment, the condition is a mood disorder.

### Brief description of the drawings

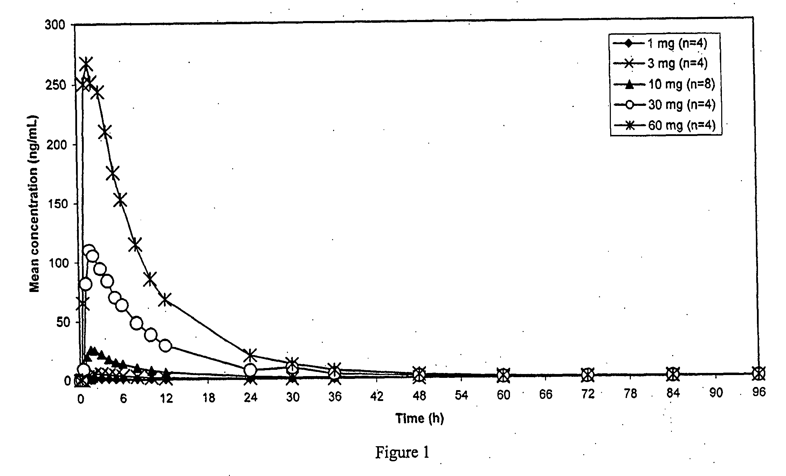

Fig. 1 is a graphical representation of the plasma concentration levels over time.

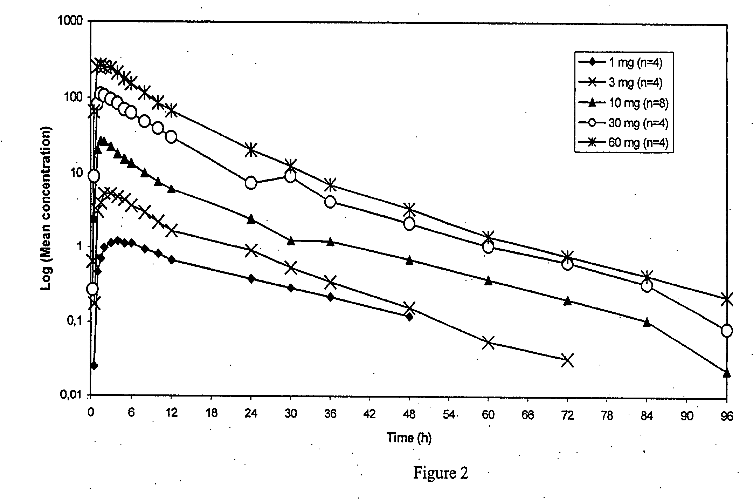

Fig. 2 is a graphical representation of the semi-logarithmic plasma concentration levels over time.
Fig. 3 is a graphical representation of a dose-normalized plot of a plasma concentration over time.

### Detailed description of the invention

The present invention provides a pharmaceutical composition useful for the treating one or more conditions or disorders in a human, which composition provides sustained therapeutic plasma level of 3α-hydroxy-3β-methoxymethyl-21-(1'-imidazolyl)-5α-pregnan-20-one. In one embodiment, the pharmaceutical formulation is a solid, oral dosage form. A condition or disorder treatable with a pharmaceutical composition of the present invention can be selected from anxiety, depression, alcohol addiction, alcohol dependence, premenstrual tension, premenstrual syndrome and premenstrual dysphoric disorder, among others. Anxiety includes, for example, generalized anxiety disorder, panic disorder, obsessive-compulsive disorder, post-traumatic stress disorder, social anxiety disorder and the like.

Clinical studies suggest that 3α-hydroxy-3β-methoxymethyl-21-(1'-imidazolyl)-5α-pregnan-20-one has the following pharmacokinetic properties in humans following oral dosing: (1) rapid absorption with Tₘₐₓ ranging from about 1 to about 3 hours; (2) variable Cₘₐₓ levels between subjects; (3) greater than dose-proportional Cₘₐₓ values; and (4) T_{1/2} values that averaged approximately 12 hours across five different dosing groups. See Table I, below.. In the context of the present invention, pharmacokinetic parameters such as AUC, Cₘₐₓ and tₘₐₓ refer to mean values. The values reported in brackets correspond to standard deviations.

**Table I**

| **Oral Dose (Fasted)** | **N** | **Tₘₐₓ (h)** | **Cₘₐₓ (ng/mL)** | **AUC (0-24) (ng/mL*h)** | **T_{1/2} (h)** | **AUC (0-last) (ng/mL*h)** |
|---|---|---|---|---|---|---|
| 1 mg | 4 | 3.1 (1.7) | 1.3 (0.3) | 16.9 (2.8) | 13.8 (0.5) | 22.4 (3.2) |
| 3 mg | 4 | 2.8 (1.0) | 5.7 (1.7) | 54.6 (23.5) | 8.8 (2.7) | 65.6 (34.7) |
| 10 mg | 8 | 1.7 (0.6) | 26.5 (10.2) | 213 (87.0) | 13.1 (3.2) | 254 (115) |
| 30mg | 4 | 2.1 (1.3) | 120(27.8) | 952(142) | 13.1 (1.0) | 1117 (185) |
| 60 mg | 4 | 1.8 (0.9) | 330 (109) | 2330 (767) | 12.5 (0.69) | 2608 (943) |

Given these pharmacokinetic properties, it is believed that compositions capable of providing plasma levels of 3α-hydroxy-3β-methoxymethyl-21-(1'-imidazolyl)-5α-pregnan-20-one in a defined therapeutic range will be beneficial in treating disorders that require sustained therapeutic plasma levels of the drug, such as mood disorders and the like. These sustained plasma levels may be effected using appropriate technologies, *e.g*., controlled-release formulations. It is believed that such formulations will confer advantages over immediate-release formulations, such as sustained efficacy, reduced side effects and improved ease of dosing.

The concentration gradients or blood plasma curves can be described by the parameters such as Cₘₐₓ, tₘₐₓ and AUC. These parameters are important in describing the pharmacokinetic properties of a specific drug formulation. Parameters describing the blood plasma curve can e.g. be obtained in clinical trials, by administration of the active agent such as 3α-hydroxy-3β-methoxymethyl-21-(1'-imidazolyl)-5α-pregnan-20-one to a number of suitable test subjects. The blood plasma values of the individual test persons are then averaged, e.g. a mean AUC, Cₘₐₓ and tₘₐₓ value is obtained. In the context of the present invention, pharmacokinetic parameters such as AUC, Cₘₐₓ and tₘₐₓ refer to mean values.

If pharmacokinetic parameters such as mean tₘₐₓ, Cₘₐₓ and AUC are measured for healthy human subjects, they are typically obtained by measuring the development of blood plasma values over time in a suitable test population of healthy human subjects.

Blood samples of the subjects may be taken at any suitable time intervals, preferably at any one or a combination of the following time points: pre-dose, 0.25, 0.5, 1.0, 1.5, 2, 3, 4, 5, 6, 8, 10, 12, 24, 30, 36, 48, 60, 72, 84 hrs post-dosing, and at End of Study, which may be e.g. at 96 h after administration of the dose.

The term "healthy" human subject in this context refers to a typical male or female of usually Caucasian, Black, Asian or other specified origin with average values as regards height, weight and physiological parameters such as blood pressure etc. Healthy human subjects for the purposes of the present invention are selected according to inclusion and exclusion criteria which are based on and in accordance with recommendations of the International Conference for Harmonization of Clinical Trials (ICH). For the purposes of the present invention, healthy subjects may be identified according to the inclusion and exclusion criteria as outlaid in the Examples.

Thus, inclusion criteria comprise an age between >21 and <45 years; a body weight ranging form 40 to 100 kg for males and a body mass index (BMI) <30 kg/m²; generally good health, evidenced by a lack of significantly abnormal findings on medical history, physical examination, vital signs, laboratory evaluations, 12-lead electrocardiogram (ECG), and ambulatory ECG using Holter-monitor for a period of 24 hours. Further, the subject preferably sleeps 6.5-8.5 hours nightly in the 30 days prior to randomization, preferably within a given time frame.

Typical exclusion parameter comprise any history of clinically significant sleep abnormality during the past 6 months; any significant sleep irregularity in the 30 days prior to randomization including night or shift work; travelling across ≥3 time zones in the 30 days prior to randomization; oxygen saturation (SpO₂) ≤94% as measured by pulse oximetry; routine daytime naps (≥15 minutes) in the 30 days prior to randomization; any history of hypersensitivity to psychotropic, or hypnotic drugs; any history of psychiatric disorders such as psychosis, obsessive-compulsive disorder, major depression or anxiety/panic disorders; a history or any current condition that might interfere with drug absorption, distribution, metabolism or excretion; use of any hypnotic or sleep aids, including melatonin, within 30 days prior to randomization; a history of drug or alcohol abuse; a history of seizures, or closed head injury in the past year; a history of smoking or use of nicotine containing products within the last three months; consumption of alcoholic beverages within forty-eight (48) hours prior to randomization; routine consumption of ≥5 cups of tea, coffee, or soda daily in the 30 days prior to randomization; consumption of caffeine-containing food or beverages during the 3 days before dosing; any clinically significant illness during the 30 days prior to randomization; any medication, including prescription and over-the-counter medications, any vitamins and/or mineral supplements that exceed 300% Daily Values, grapefruit juice, and St. John's Wort during the seven (7) days prior to randomization; refusage to abstain from food ten (10) hours preceding dosing and for four (4) hours following study drug administration and refuse to abstain from alcohol, caffeine or xanthine-containing food or beverages for the entire study period; participation in a clinical study in the 30 days prior to randomization; blood or blood product donation in the 30 days prior to randomization; positive results of urine drug screen, urine cotinine, blood alcohol, HBₛAg, HBₛAb (unless subject has been immunized), anti-HCV, or anti-HIV; sleep latency >30 minutes, or sleep efficiency ≤85% or >95%, as indicated by the sleep log from Day -7 to Day -1, or by the actigraphy on the night of Day -1 (first night after checking in).

Ifpharmacokinetic parameters such as mean tₘₐₓ, Cₘₐₓ and AUC are obtained in subjects, the subject group will comprise a suitable number of subjects. A reasonable number of subjects will e.g. be 4, 8, 10, 20, 30, 40, 50, 60, 72, or even more patients. Subjects will be selected according to symptoms of the condition to be treated. The subjects may be grouped into cohorts of suitable sizes e.g. for examination of dose escalation. For the purposes of the present invention, subjects may be selected according to the inclusion and exclusion criteria provided within the Examples.

It is to be understood that values of pharmacokinetic parameters as indicated above and below have been deduced on the basis of the data which were obtained in Example 7, all of which relate to single dose studies in healthy human subjects. However, it is assumed that comparable results will be obtained upon steady state administration in healthy human subject or single dose and steady state administration in human patients.

The pharmaceutical composition according to the present invention exhibits Tₘₐₓ- value in the range of about 1.5 to about 5 hours, preferably in the range of about 1.3 to about 3.5 h and more preferably in the range of about 1.6 to about 3.2 h. Clinical studies suggest that the compositions according to the present invention are rapidly absorbed, preferably independent from the amount of active agent, i.e. 3α-hydroxy-3β-methoxymethyl-21-(1'-imidazolyl)-5α-pregnan-20-one present in the pharmaceutical composition according to the present invention.

The Cₘₐₓ value of the pharmaceutical composition of the present invention is in the range of about 1.0 ng/mL to about 1.8 ng/mL, preferably in the range of about 1.2 ng/mL to about 1.6 ng/mL for a dosage form comprising 1 mg 3α-hydroxy-3β-methoxymethyl-21-(1'-imidazolyl)-5α-pregnan-20-one.

The Cₘₐₓ value of the pharmaceutical composition of the present invention is in the range of about 4.0 ng/mL to about 8.0 ng/mL, preferably in the range of about 4.0 ng/mL to about 6.5 ng/mL, more preferably from about 5.0 ng/mL to about 6.0 ng/mL for a dosage form comprising 3 mg 3α-hydroxy-3β-methoxymethyl-21-(1'-imidazolyl)-5α-pregnan-20-one.

The Cₘₐₓ value of the pharmaceutical composition of the present invention is in the range of about 16.9 ng/mL to about 46.3 ng/mL, preferably in the range of about 21.0 ng/mL to about 32.0 ng/mL, more preferably from about 25.0 ng/mL to about 28.0 ng/mL for a dosage form comprising 10 mg 3α-hydroxy-3β-methoxymethyl-21-(1'-imidazolyl)-5α-pregnan-20-one.

The Cₘₐₓ value of the pharmaceutical composition of the present invention is in the range of about 95.6 ng/mL to about 154.0 ng/mL, preferably in the range of about 108.0 ng/mL to about 134.0 ng/mL, more preferably in the range of about 115.0 ng/mL to about 125.0 ng/mL for a dosage form comprising 30 mg 3α-hydroxy-3β-methoxymethyl-21-(1'-imidazolyl)-5α-pregnan-20-one.

The Cₘₐₓ value of the pharmaceutical composition of the present invention is in the range of about 218.0 ng/mL to about 479.0 ng/mL, preferably in the range of about 275.0 ng/mL to about 385.0 ng/mL, more preferably from about 300 ng/mL to about 350 ng/mL for a dosage form comprising 60 mg 3α-hydroxy-3β-methoxymethyl-21-(1'-imidazolyl)-5α-pregnan-20-one.

Further, by the administration of the pharmaceutical compositions according to the present invention, greater than dose proportional Cₘₐₓ values may be obtained.

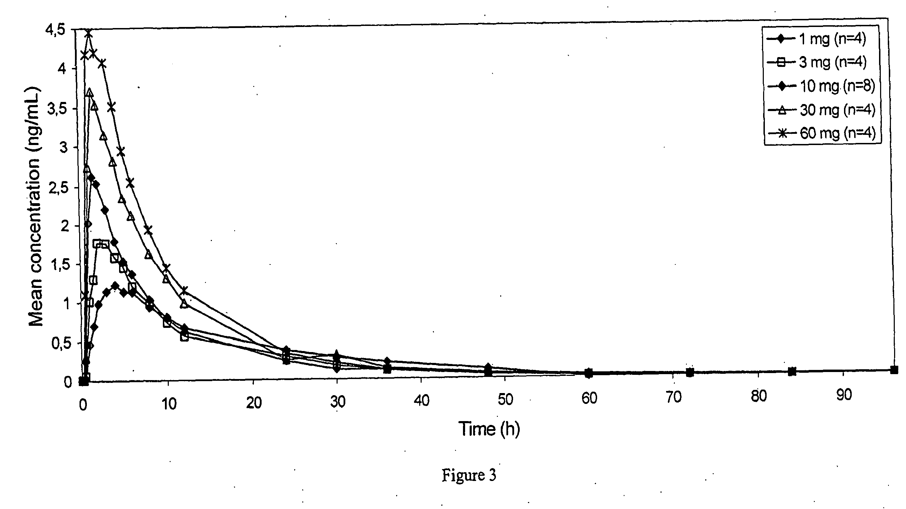

Greater than dose proportional Cₘₐₓ value in the context of the present invention means that the active agent normalized Cₘₐₓ value is not constant. In other words, the normalized Cₘₐₓ for an amount 1, e.g. 10 mg, of active agent is greater than the normalized Cₘₐₓ for an amount 2, e.g. 1 mg of active agent. The greater than dose proportional Cₘₐₓ behaviour of the active agent 3α-hydroxy-3β-methoxymethyl-21-(1'-imidazolyl)-5α-pregnan-20-one is also shown in Fig. 3. Fig. 3 shows a dose normalized plasma concentration vs. time diagram. The normalized Cₘₐₓ values for the 3 mg, 10 mg, 30 mg and 60 mg dosage form according to the present invention comprising 3α-hydroxy-3β-methoxymethyl-21-(1'-imidazolyl)-5α-pregnan-20-one are greater than the Cₘₐₓ obtained for the 1 mg dosage form according to the present invention. The normalized Cₘₐₓ values obtained for the dosage forms comprising more than 1 mg acitive agent may have normalized Cₘₐₓ values which are at least about 2, at least about 2.5, at least about 3, at least about 4, at least about 5 times greater than the Cₘₐₓ value obtained from the 1 mg dosage form according to the present invention. It can be assumed that dosage forms which comprise more than 60 mg of 3α-hydroxy-3β-methoxymethyl-21-(1'-imidazolyl)-5α-pregnan-20-one exhibits dose proportional Cₘₐₓ values greater than at least about 5. Without being bound to any theory, it can be assumed that normalized Cₘₐₓ values of greater than at least about 7, at least about 10, at least about 15 or at least about 20 or at least about 25 may be achieved with the dosage forms of the present invention compared to the Cₘₐₓ value of the 1 mg dosage form according to the present invention.

The greater than dose proportional Cₘₐₓ values of the pharmaceutical compositions according to the present invention may allow for a significant increase of Cₘₐₓ while the amount of active agent is only slightly varied or maintained almost at the same level.

Therapeutic plasma levels of 3α-hydroxy-3β-methoxymethyl-21-(1'-imidazolyl)-5α-pregnan-20-one can range from about 5 ng/mL to about 500 ng/mL. Other therapeutic ranges of 3α-hydroxy-3β-methoxymethyl-21-(1'-imidazolyl)-5α-pregnan-20-one include from about 50 ng/mL to about 500 ng/mL, from about 50 ng/mL to about 400 ng/mL, from about 50 ng/mL to about 325 ng/mL, from about 50 ng/mL to about 250 ng/mL, from about 50 ng/mL to about 100 ng/mL, and from about 100 ng/mL to about 250 ng/mL.

These steady state plasma levels can be effected using appropriate technologies, *e.g*., controlled-release formulations having an appropriate release profile. The appropriate release profile can be achieved, for example, using single or multiparticulate delivery systems. Examples of single delivery systems include, but are not limited to, wax matrix tablets, hydrophilic matrix tablets and tablets with controlled-release coatings. Examples of multiparticulate systems include, but are not limited to, matrix systems such as Melt Extruded Multiparticulates or systems based on controlled release coatings such as coated-beads.

These controlled-release pharmaceutical compositions according to the present invention may include a controlled-release a controlled release material which is incorporated into the matrix along with the active agent, or which is applied as a controlled release coating over a substrate comprising the active agent. The term "substrate" may include beads, pellets, spheroids, tablets, tables cores, etc. The controlled release material may be hydrophobic or hydrophilic as desired. The dosage forms according to the present invention, preferably oral dosage forms, may be provided e.g. as granules, spheroids, pellets, etc. On the other hand, the dosage form of the present invention may be prepared as a tablet core coated with a controlled release coating or as a tablet comprising a matrix of the active agent, controlled release material, and optionally other pharmaceutically acceptable ingredients such as binders, diluents, colorants, lubricants, etc.

Examples for controlled release formulation may include formulation disclosed in WO01/32148, US 4,861,598, US 4,990,341, US 4,884,909, references cited therein and others discloed in the art.

In one embodiment, the pharmaceutical compositions of the present invention provide therapeutic steady state plasma levels of 3α-hydroxy-3β-methoxymethyl-21-(1'-imidazolyl)-5α-pregnan-20-one for a duration of from about 12 h to about 24 h following administration. In another embodiment, the pharmaceutical compositions of the present invention provide therapeutic steady state plasma levels of 3α-hydroxy-3β-methoxymethyl-21-(1'-imidazolyl)-5α-pregnan-20-one for a duration of from about 6 h to about 12 h following administration. 3α-Hydroxy-3β-methoxymethyl-21-(1'-imidazolyl)-5α-pregnan-20-one may exist as optical isomers (enantiomers), and the invention includes both racemic mixtures and enantiomerically-enriched mixtures of such optical isomers, as well as the individual entantiomers that may be separated according to methods that are well known to those of ordinary skill in the art.

3α-Hydroxy-3β-methoxymethyl-21-(1'-imidazolyl)-5α-pregnan-20-one may exist as isomorphic crystalline habits as described in U.S. Patent Appl. No. 60/604,447, which is hereby incorporated by reference in its entirety.

Also included within the scope of the present invention are compositions that provide steady state therapeutic plasma levels of 3α-hydroxy-3β-methoxymethyl-21-(1'-imidazolyl)-5α-pregnan-20-one that comprise solvated forms of 3α-hydroxy-3β-methoxymethyl-21-(1'-imidazolyl)-5α-pregnan-20-one, especially hydrated forms. Hydration may occur during manufacturing of 3α-hydroxy-3β-methoxymethyl-21-(1'-imidazolyl)-5α-pregnan-20-one or compositions comprising 3α-hydroxy-3β-methoxymethyl-21-(1'-imidazolyl)-5α-pregnan-20-one, or the hydration may occur over time due to the hygroscopic nature of 3α-hydroxy-3β-methoxymethyl-21-(1'-imidazolyl)-5α-pregnan-20-one. Also included within the scope of the present invention are compositions that provide steady state therapeutic plasma levels of 3α-hydroxy-3β-methoxymethyl-21-(1'-imidazolyl)-5α-pregnan-20-one that comprise pharmaceutically-acceptable salts of 3α-hydroxy-3β-methoxymethyl-21-(1'-imidazolyl)-5α-pregnan-20-one. Pharmaceutically-acceptable acid addition salts are formed by mixing a solution of 3α-hydroxy-3β-methoxymethyl-21-(1'-imidazolyl)-5α-pregnan-20-one with a solution of a pharmaceutically-acceptable non-toxic acid such as hydrochloric acid, fumaric acid, maleic acid, succinic acid, acetic acid, citric acid, tartaric acid, carbonic acid, phosphoric acid, oxalic acid, dichloroacetic acid and the like. Pharmaceutically-acceptable basic salts are formed by mixing a solution of 3α-hydroxy-3β-methoxymethyl-21-(1'-imidazolyl)-5α-pregnan-20-one with a solution of a pharmaceutically-acceptable non-toxic base such as sodium hydroxide, potassium hydroxide, choline hydroxide, sodium carbonate and the like.

The present invention also relates to a method of treating a condition or disorder which treatment benefits from sustained therapeutic plasma levels of 3α-hydroxy-3β-methoxymethyl-21-(1'-imidazolyl)-5α-pregnan-20-one. The method of treating comprises administering to a subject in need of such treatment a pharmaceutical composition that provides steady state therapeutic plasma levels of 3α-hydroxy-3β-methoxymethyl-21-(1'-imidazolyl)-5α-pregnan-20-one in accordance with the present invention. Such conditions and disorders include mood disorders such as anxiety, depression, alcohol addiction and/or dependence, premenstrual tension, premenstrual syndrome, premenstrual dysphoric disorder and the like. Anxiety includes, for example, generalized anxiety disorder, panic disorder, obsessive-compulsive disorder, post-traumatic stress disorder, social anxiety disorder and the like.

### Definitions

The term "treating" or "treatment" of a condition or disorder as employed herein refers to (i) inhibiting the condition or disorder, *i.e.,* arresting the development of the condition or disorder or its clinical symptoms, and/or (ii) relieving the condition or disorder, i.e., causing temporary or permanent regression of the condition or disorder or its clinical symptoms.

The term "chiral center" refers to a carbon atom to which four different groups are attached, or a sulfur atom to which three different groups are attached, where the sulfur atom and its attached groups form a sulfoxide, sulfinic ester, sulfonium salt or sulfite.

The term "enantiomer" or "enantiomeric" refers to a molecule that is nonsuperimposable on its mirror image and hence optically active wherein the enantiomer rotates the plane of polarized light in one direction and its mirror image rotates the plane of polarized light in the opposite direction.

The term "racemic" refers to a mixture of equal parts of enantiomers and which is optically inactive.

The term "resolution" refers to the separation or concentration or depletion of one of the two enantiomeric forms of a molecule. The phrase "enantiomerically enriched" refers to a mixture in which one enantiomer is present in a greater concentration than its mirror image molecule.

The Cₘₐₓ value indicates the maximum blood plasma concentration of the active agents, i.e. 3α-hydroxy-3β-methoxymethyl-21-(1'-imidazolyl)-5α-pregnan-20-one.

The tₘₐₓ value indicates the time point at which the Cₘₐₓ value is reached. In other words, tₘₐₓ is the time point of the maximum observed plasma concentration.

The AUC (Area Under the Curve) value corresponds to the area of the concentration curve. The AUC value is proportional to the amount of active agents, i.e. 3α-hydroxy-3β-methoxymethyl-21-(1'-imidazolyl)-5α-pregnan-20-one absorbed into the blood circulation in total and is hence a measure for the bioavailability.

The AUC(0-24) value is the value for the area under the plasma concentration-time curve from the time of administration to until 24 h after administration.

The AUC (0 to last) value is the value for the area under the plasma concentration-time curve from the time of administration to the last measurable concentration.

The term "normalized Cₘₐₓ" refers to a ratio of the Cₘₐₓ value of a specific dosage of the active agent and the amount of active agent.

The term "bioavailability" is defined for purposes of the present invention as the extent to which active agents such as 3α-hydroxy-3β-methoxymethyl-21-(1'-imidazolyl)-5α-pregnan-20-one are absorbed from the dosage forms.

The term "mean" in the context of the present invention refers the mean of the data of at least two subjects.

The term "sustained release" is defined for purposes of the present invention as the release of 3α-hydroxy-3β-methoxymethyl-21-(1'-imidazolyl)-5α-pregnan-20-one at such a rate that blood levels are maintained within the therapeutic range but below toxic levels over a period of time of about 6 hours or about 12 hours or about 24 hours or even longer. The term "sustained release" differentiates the preparations in accordance with the invention from "immediate release" preparations. The terms "sustained release" and "controlled release" are used interchangeably.

The term t_{1/2} is defined for purposes of the present invention as the amount of time necessary for one half of the absorbable dose of 3α-hydroxy-3β-methoxymethyl-21-(1'-imidazolyl)-5α-pregnan-20-one to be transferred to plasma. This value may be calculated as a "true" value (which would take into account the effect of elimination processes), rather than an "apparent" absorption half-life.

The term "steady state" means that a plasma level for a given drug has been achieved and which is maintained with subsequent doses of the drug at a level which is at or above the minimum effective therapeutic level and is below the minimum toxic plasma level for 3α-hydroxy-3β-methoxymethyl-21-(1'-imidazolyl)-5α-pregnan-20-one.

The minimum effective therapeutic level will be partially determined by the extend of the desired effect achieved in a given patient. It will be well understood by those skilled in the medical art that this measurement is highly subjective and great individual variations may occur among subjects. It is clear that after the administration of each dose the concentration passes through a maximum and then again drops to a minimum.

The steady state may be described as follows: At the time t = 0, the time the first dose is administered, the concentration C is also 0. The concentration then passes through a first maximum and then drops to a first minimum. Before the concentration drops to 0, another dose is administered, so that the second increase in concentration doesn't start at 0. Building on this first concentration minimum, the curve passes through a second maximum after the second dose has been administered, which is above the first maximum, and drops to a second minimum, which is above the first minimum. Thus, the blood plasma curve escalates due to the repeated doses and the associated step-by-step accumulation of active agent, until it levels off to a point where absorption and elimination are in balance. This state, at which absorption and elimination are in equilibrium and the concentration oscillates constantly between a defined minimum and a defined maximum, is called steady state.

The pharmaceutical compositions of the present invention can be administered by any means that achieve their intended purpose. For example, administration can be by subcutaneous, intravenous, intramuscular, intraperitoneal, buccal, or ocular routes, rectally, parenterally, intrasystemically, intravaginally, topically (as by powders, ointments, drops or transdermal patch), or as an oral or nasal spray. Alternatively, or concurrently, administration can be by the oral route. Administration of the pharmaceutical compositions of the present invention by the oral route is currently preferred.

Frequency of administration should be appropriate for the duration of action anticipated for the administered composition. For example, for a composition that provides therapeutic plasma levels of 3α-hydroxy-3β-methoxymethyl-21-(1'-imidazolyl)-5α-pregnan-20-one for a duration of from about 12 h to about 24 h following administration, the composition may be given once per day. Similarly, for a composition that provides therapeutic plasma levels of 3α-hydroxy-3β-methoxymethyl-21-(1'-imidazolyl)-5α-pregnan-20-one for a duration of from about 6 h to about 12 h following administration, the composition may be given twice per day.

The following examples illustrate, but do not limit, the compositions and methods of the present invention. Other suitable modifications and adaptations of the variety of conditions and parameters normally encountered in clinical therapy and which are obvious to those skilled in the art are within the spirit and scope of the invention.

### Examples

3α-Hydroxy-3β-methoxymethyl-5α-pregnan-20-one can be prepared from (3R)-spiro[oxirane-2α, 5α-pregnan]-20-one and sodium methoxide as described by Hogenkamp, et al., "Synthesis and in Vitro Activity of 3β-Substituted-3α-hydroxypregnan-20-ones: Allosteric Modulators of the GABAA Receptor," J. Med. Chem. 40:61-72 (1997).

Based on single-dose trials of 3α-hydroxy-3β-methoxymethyl-21-(1'-imidazolyl)-5α-pregnan-20-one, where dosing was with immediate- release suspensions, a dose of approximately 30 mg (based on the free compound) given once daily is expected to be appropriate for a composition that provides therapeutic plasma levels of 3α-hydroxy-3β-methoxymethyl-21-(1'-imidazolyl)-5α-pregnan-20-one for a duration of from about 12 h to about 24 h following administration. This amount is used in the following examples, but it is understood that the dose needed could range from about 20 mg/day to about 40 mg/day. If the 3α-hydroxy-3β-methoxymethyl-21-(1'-imidazolyl)-5α-pregnan-20-one is in the form of a salt, appropriate adjustments to the amount may be made. Such modifications are well within the knowledge and capabilities of one of skill in the art.

The dosage can be adjusted to attain a desired duration of action. For example, a dose of approximately 15 mg (based on the free compound) given twice daily is expected to be appropriate for a composition that provides therapeutic plasma levels of 3α-hydroxy-3β-methoxymethyl-21-(1'-imidazolyl)-5α-pregnan-20-one for a duration of from about 6 h to about 12 h following administration.

### Example 1

Preparation of 3α-hydroxy-3β-methoxymethyl-21-(1'-imidazolyl)-5α-pregnan-20-one

The title compound and its hydrochloride salt may be prepared as follows.
a) *21-Bromo-3*α*-hydroxy-3β-methoxymethyl-5*α*-pregnan-20-one.* To a solution of 3α-hydroxy-3β-methoxymethyl-5α-pregnan-20-one (30.0 g, 82.9 mmol) in about 900 mL of methanol stirring at rt is added 3 drops of a 48% aqueous HBr solution. Bromine (13.9 g, 87.1 mmol) is then added dropwise as a solution in about 200 mL of methanol over about 2 h during which the reaction is shielded from light. TLC (1% acetone/CH₂Cl₂) may be used to indicate the absence of starting material and the formation of a less polar product (after about an additional 30 min). The reaction is concentrated to approximately 300 mL. CH₂Cl₂ (about 400 mL) is added and the reaction is poured into a separatory funnel containing about 200 mL of water. The phases are separated and the aqueous phase is extracted with CH₂Cl₂ (about 100 mL, 3x). The organic phases are combined, washed with about 200 mL of a saturated aqueous NaHCO₃ solution, dried over Na₂SO₄ and concentrated under reduced pressure to afford the bromide as a pale yellow foam. The product may be used in the next step without further purification.
b) *3*α*-Hydroxy-3β-methoxymethyl-21-(1'-imidazolyl)-5*α-*pregnan-20-one.* To a suspension of the bromide prepared as above (36.7 g, 82.9 mmol) in about 800 mL of CH₃CN is added imidazole (28.2 g, 415 mmol), and the reaction is heated to reflux under Ar. TLC (95:4.5:0.5 CH₂Cl₂:MeOH:triethylamine) may be used to indicate completion of the reaction (after about 1 hour at reflux). The reaction is cooled to room temperature and is concentrated in vacuo. The resulting oil is dissolved in about 600 mL of CH₂Cl₂, washed with a dilute NaHCO₃ solution (about 200 mL, 4x), dried over Na₂SO₄ and concentrated in vacuo. Purification via flash chromatography on silica gel eluting with 95:4.5:0.5 CH₂Cl₂:MeOH:triethylamine affords about 18 g of the title compound as a white solid, m.p. 185-187°C (approx.). Anal. calcd. for C₂₆H₄₀N₂O₃: C, 72.86; H, 9.41; N, 6.54. ¹H NMR (300 MHz, CDCl₃) δ (approx.) 7.40 (s, 1 H), 7.08 (s, 1H), 6.84 (s, 1H), 4.72 (d, 1H, J=17.7 Hz), 4.64 (d, 1H, J=18 Hz), 3.39 (s, 3H), 3.18 (s, 2H), 2.57 (t, 1H, J=8.7 Hz), 0.76 (s, 3H), 0.66 (s, 3H).
c) *3*α-*Hydroxy-3β-methoxymethyl-21-(1'-imidazolyl)-5*α-*pregnan-20-one, hydrochloride salt.* Hydrochloric gas is bubbled through a solution of 3α-hydroxy-3β-methoxymethyl-21-(1'-imidazolyl)-5α-pregnan-20-one (1.00 g, 2.33 mmol dissolved in about 35 mL of CH₂Cl₂) for about 7 min. A white precipitate forms. The solvent is removed in vacuo to afford about 1.10 g of the hydrochloride salt as a white solid, m.p. 230-233°C (approx.). ¹H NMR (300 MHz, CDCl₃) δ (approx.) 9.66 (s, 1 H), 7.31 (s, 1 H), 7.05 (s, 1 H), 5.45 (d, 1 H, J=18 Hz), 5.26 (d, 1 H, J=18 Hz), 3.39 (s, 3H), 3.19 (s, 2H), 2.72 (t, 1 H, J=8.7 Hz), 0.76 (s, 3H), 0.70 (s, 3H).

### Example 2

Hydrophilic matrix tablet comprising 3α-hydroxy-3β-methoxymethyl-21-(1'-imidazolyl)-5α-pregnan-20-one

| | |
|---|---|
| 3α-Hydroxy-3β-methoxymethyl-21-(1'-imidazolyl)-5α-pregnan-20-one | 30 mg |
| Hydroxypropylmethyl cellulose | 30 mg |
| Spray-dried lactose | 88.9 mg |
| Colloidal silicon dioxide | 0.15 mg |
| Magnesium stearate | 1 mg |
| Total | 150 mg |

Blend the colloidal silicon dioxide and a fraction of the lactose. Screen the mixture. Add the 3α-hydroxy-3β-methoxymethyl-21-(1'-imidazolyl)-5α-pregnan-20-one, hydroxypropylmethylcellulose, and the remaining lactose to the mixture and blend. Add the magnesium stearate and blend. Compress the final blend to a target weight of 150 mg.

### Example 3

Encapsulated Melt Extruded Multiparticulates (MEMs) comprising 3α-hydroxy-3β-methoxymethyl-21-(1'-imidazolyl)-5α-pregnan-20-one

| | |
|---|---|
| 3α-Hydroxy-3β-methoxymethyl-21-(1'-imidazolyl)-5α-pregnan-20-one | 30 mg |
| Eudragit RLPO | 50 mg |
| Eudragit RSPO | 132 mg |
| Stearyl Alcohol | 28 mg |
| Glyceryl behenate | 10 mg |
| Total | 250 mg |

| | |
|---|---|
| Size #1 Hard Gelatin Capsule shell. | |

Blend the 3α-hydroxy-3β-methoxymethyl-21-(1'-imidazolyl)-5α-pregnan-20-one, Eudragit RSPO, Eudragit RLPO, stearyl alcohol and glyceryl behenate. Extrude using a hot-melt extruder into strands of approximately 1 mm diameter, and cut the strands into lengths of approximately 1 mm to create the MEMs. Encapsulate the MEMs into hard-gelatin capsules at a target fill weight of 250 mg.

### Example 4

Hydrophobic matrix tablet comprising 3α-hydroxy-3β-methoxymethyl-21-(1'-imidazotyl)-5α-pregnan-20-one

| | |
|---|---|
| 3α-Hydroxy-3β-methoxymethyl-21-(1'-imidazolyl)-5α-pregnan-20-one | 30 mg |
| Spray-dried Lactose | 70 mg |
| Hydroxyethylcellulose | 10 mg |
| Cetostearyl Alcohol | 25 mg |
| Talc | 3 mg |
| Magnesium stearate | 2 mg |
| Total | 140 mg |

Wet-granulate the 3α-hydroxy-3β-methoxymethyl-21-(1'-imidazolyl)-5α-pregnan-20-one, lactose and cetostearyl alcohol using the hydroxyethylcellulose as a binder. Blend the dried and screened granulation with the talc. Add the magnesium stearate and blend. Compress to a target weight of 140 mg.

### Example 5

Melt-Extruded Granulation (MEG) tablet comprising 3α-hydroxy-3β-methoxymethyl-21-(1'-imidazolyl)-5α-pregnan-20-one

| | |
|---|---|
| 3α-Hydroxy-3β-methoxymethyl-21-(1'-imidazolyl)-5α-pregnan-20-one | 30 mg |
| Stearyl Alcohol | 20 mg |
| Glyceryl behenate | 10 mg |
| Dibasic Calcium Phosphate | 29 mg |
| Microcrystalline Cellulose | 30 mg |
| Magnesiun stearate | 1 mg |
| Total | 120 mg |

Blend the 3α-hydroxy-3β-methoxymethyl-21-(1'-imidazolyl)-5α-pregnan-20-one, stearyl alcohol, glyceryl behenate and approximately half of the dibasic calcium phosphate. Extrude the blend into strands several mm in diameter, at a temperature sufficient to melt the stearyl alcohol and glyceryl behenate. Cut the strands into lengths several mm long. Mill the resultant sections of extrudate using a high-speed mill. Blend the milled extrudate with the remaining dibasic calcium phosphate and the microcrystalline cellulose. Add the magnesium stearate and blend. Compress to a target weight of 120 mg.

### Example 6

Controlled release beads comprising 3α-hydroxy-3β-methoxymethyl-21-(1'-imidazolyl)-5α-pregnan-20-one

| **Formula** | **Ingredients** | **Amt/Unit (mg)** | **Amt/Batch(g)** |
|---|---|---|---|
| Step 1. Drug Layering | 3α-Hydroxy-3β-methoxymethyl-21-(1'-imidazolyl)-5α-pregnan-20-one | 30.0 | 112.5 |
| | Nu Pariel beads | 150.0 | 562.5 |
| | Opadry Clear | 1.5 | 5.6 |
| | Water | qs | 563 mL |
| Step 2. Enteric coat | Eudragit L30D-55 (solids) | 20 | 75 |
| | Triethyl Citrate | 4 | 15 |
| | Talc | 4.5 | 16.9 |
| | Water | qs | 240mL |
| Total | | 210 mg | 787.5 g |

Step 1. Dissolve the 3α-hydroxy-3β-methoxymethyl-21-(1'-imidazolyl)-5α-pregnan-20-one in 563 mL of water in a vessel using a mixer. Then dissolve the Opadry Clear into this solution. In a 1 kg fluid bed, spray the above solution onto the NuPariel beads at about 10 mL/minute at an inlet temperature of about 40°C

Step 2. Disperse the Triethylcitrate and talc into the water then add the Eudragit L30D dispersion using a mixer. When dispersed, spray this dispersion onto the drug loaded beads from Step 1 in a 1 kg fluid bed at about 10 ml/minute at an inlet temperature of about 40°C.

### Example 7

To determine the overall effect and the pharmacokinetic parameters of the pharmaceutical composition according to the invention, an escalating single-dose, randomized, double blind, placebo-controlled study in healthy male subjects has been performed. The pharmaceutical composition according to the present invention has been administered as an oral suspension.

The anticipated initial dose escalation sequence is: 1, 3, 10, 30, 100, 300, 600 and 1000 mg. Further, a 60 mg dose of the active agent has bee tested.

Test Population, Inclusion and Exclusion Criteria

A total number of up to 72 subjects in groups of 6 subjects were to be enrolled in the clinical phase I study in the USA.

Study participants were selected according to the inclusion and exclusion criteria listed below. In general, healthy male subjects, aged in the range of 21 to 45 years which revealed a normal sleep history are suitable subjects for the present study.

In particular, subjects were selected according to the following inclusion criteria:
Male subjects 21 to 45 years of age.
   - With body weight ranging from 40 to 100 kg and a Body Mass Index (BMI) <30 kg/m2.
   - Who are healthy as determined by unremarkable medical history, physical examination, vital signs, laboratory evaluations, 12-lead electrocardiogram (ECG), and ambulatory ECG using Holter-monitor for a period of 24 hours.
   - Who sleep 6.5-8.5 hours nightly in the 30 days prior to randomization. Nightly bedtime routine is between 9:30 PM and 12:00 AM for a minimum of one week prior to study entry.
   - Who sign the informed consent prior to beginning protocol-specific procedures.
Subjects which were excluded from the present study were those:
   - With any history of clinically significant sleep abnormality during the past 6 months.
   - With any significant sleep irregularity in the 30 days prior to randomization including night or shift work.
   - Who travel across ≥3 time zones in the 30 days prior to randomization.
   - With oxygen saturation (SpO2) <94% as measured by pulse oximetry.
   - Who take routine daytime naps (≥15 minutes) in the 30 days prior to randomization.
   - With any history of hypersensitivity to psychotropic, or hypnotic drugs.
   - With any history of psychiatric disorders such as psychosis, obsessive-compulsive disorder, major depression or anxiety/panic disorders.
   - With a history or any current condition that might interfere with drug absorption, distribution, metabolism or excretion.
   - Who use any hypnotic or sleep aids, including melatonin, within 30 days prior to randomization.
   - With a history of drug or alcohol abuse.
   - With a history of seizures, or closed head injury in the past year.
   - With a history of smoking or use of nicotine containing products within the last three months.
   - Who consume alcoholic beverages within forty-eight (48) hours prior to randomization.
   - Who routinely consume ≥5 cups of tea, coffee, or soda daily in the 30 days prior to randomization.
   - Who consume caffeine-containing food or beverages during the 3 days before dosing.
   - With any clinically significant illness during the 30 days prior to randomization.
   - Who use any medication, including prescription and over-the-counter medications, any vitamins and/or mineral supplements that exceed 300% Daily Values, grapefruit juice, and St. John's Wort during the seven (7) days prior to randomization.
   - Who refuse to abstain from food ten (10) hours preceding dosing and for four (4) hours following study drug administration and refuse to abstain from alcohol, caffeine or xanthine-containing food or beverages for the entire study period.
   - Who participated in a clinical study in the 30 days prior to randomization.
   - Who donated blood or blood products in the 30 days prior to randomization.
   - Who have positive results of urine drug screen, urine cotinine, blood alcohol, HBsAg, HBsAb (unless subject has been immunized), anti-HCV, or anti-HIV.
   - With a sleep latency >30 minutes, or sleep efficiency <85% or >95%, as indicated by the sleep log from Day -7 to Day -1, or by the actigraphy on the night of Day -1 (first night after checking in).

Minor deviations from the inclusion or exclusion criteria are allowed only by special permission from the Sponsor's medical monitor prior to entry of the subject into the study.

### Study Design:

The study is designed as an escalating single-dose, randomized, double-blind, placebo-controlled study in healthy adult male subjects.

The study will be conducted in cohorts of 6 subjects, 4 of whom will be randomized to receive active drug and 2 of whom will receive placebo. Up to 12 groups may be studied.

The anticipated initial dose escalation sequence is: 1, 3, 10, 30, 100, 300, 600 and 1000 mg. This sequence may be changed, if indicated, based on the following principles: 1) An additional subject group at a previous dose level may be studied to enhance the clinical observation; 2) Reducing the escalation ratio for a subsequent cohort, including study of a dose lower than the preceding maximum dose; and 3) A dose level may be administered with food (e.g., high fat meal) to determine the influence of food on bioavailability. When administered with food, the dose will not be greater than 1/10 the previously administered, well-tolerated dose under fasting conditions. The criteria for dose escalation are safety, tolerability, and available pharmacokinetic data following administration of study drug to each cohort.

Dose escalation will continue until dose-limiting toxicity is encountered or until the maximum dose is reached.

Basically the study is designed in the following phases:
- Screening
- Baseline Period
- Treatment Period

The screening period can last for up to 28 days prior to randomization. During this period, subjects will be assessed for study eligibility and maintain a sleep log for 6 consecutive nights prior to admission to the study unit.

The following evaluations will be performed in the Screening Period after the subject signs an informed consent on or prior to Day-7.
- Medical history
- Physical examination
- Vital signs
- Oxygen saturation measured by finger pulse oximetry
- Laboratory evaluations
- Conventional 12-lead ECG
- 24 hr ECG with a Holter monitor or a H-12 Digital Recorder Inclusion and exclusion criteria

During the 24 hr period when subjects are wearing the Holter or H-12 Digital Recorder, they are restricted from vigorous physical activities or exercise.

The above mentioned laboratory evaluations include: Hematology, serum chemistry, liver function tests, serology for HBₛAg, HBₛAb, anti-HCV, and anti-HIV, urinalysis, blood alcohol, and urine drug/cotinine screening.

A preliminary evaluation on the subject's eligibility will be performed based on inclusion and exclusion criteria specified above and the results of the above examinations.

The Baseline Period is from check-in on the evening of Day -2 to immediately prior to the first pre-dose measurement on Day 1. During this period, subjects will stay at the study facility and be further assessed for their eligibility through updated medical history, physical examination, vital signs, pulse oximetry, laboratory tests, drug screening, 24 hr ECG, 24 hr EEG, overnight actigraphy, and CCT (Computer Cognitive Test) and SSS (Stanford Sleepiness Scale) training. Their sleep log from Day -1 will be completed and reviewed on the morning of Day 1. Subjects who meet entry criteria will be randomized at the end of the Baseline Period.

The Baseline period will start at admission to study unit on the evening of Day -2 and end immediately prior to the first pre-dose measurement on Day 1.

Subjects will be admitted to the study unit on Day -2 and be confined to the facility until study Day 3 (4 nights and 5 days). Meals and snacks will be provided by the study center. No food or drink is permitted to be brought into the study facility by the subjects. No grapefruit, grapefruit juice, alcohol, caffeine or xanthine containing food or beverage (including chocolate) will be allowed during the entire stay. No smoking or medication other than study drug, including over-the-counter and herbal medications, such as St. John's Wort, will be permitted. If any medication is used during the baseline period, even to treat an adverse event, the subject will be discontinued from the study.

Baseline vital signs and SpO₂ will be measured in supine position at approximately - 24, -23.75, -23.5, -23, -22.5, -22, -21, -20, -19, -18, -16, -14 and -12 hrs pre-dose after the subjects have rested in a supine position for 3 minutes.

In addition, baseline conventional 12-lead ECGs and EEG with SpO₂ will be performed.

Blood alcohol, urine drug/cotinine screen and laboratory tests (hematology, serum chemistry, liver function tests, and urinalysis, etc.) will be performed. The subjects' sleep log for the past 6 days will be reviewed for eligibility.

The treatment period will be up to 5-days in duration. In the morning of the dosing day, subjects will be randomized to receive an oral dose of either the active study drug or placebo. The subjects will be monitored closely for safety and be tested using various pharmacodynamic measures during this phase. E.g. blood and urine samples will be collected for determination of the concentration of the active agent and its metabolites.

The Double-Blind Phase starts with the collection of the first pre-dose measurement on Day 1 and continues to the End of Study on Day 5.

Pre-dose vital sign measurements, pulse oximetry and ECG will be performed in the above order within 30 minutes prior to dosing. Further, pre-dose blood sample and laboratory tests (hematology, serum chemistry, liver function tests, and urinalysis) will be collected after the vital signs, SpO₂ and ECG have been measured.

Subsequently, pre-dosing SSS (Stanford sleepiness scale) and CCT (Computerized Cognitive Tests) will be performed after the pre-dose blood sample collection. SSS will be done first, then CCT will be performed in the following sequence: Simple Reaction Time, Choice Reaction Time, and then Digit Vigilance.

After administration, vital signs and SpO₂ will be taken at 0.25, 0.5, 1.0, 1.5, 2, 3, 4, 5, 6, 8, 10, 12, 24, 30, 36, 48, 60, 72, 84 hrs after dosing, and at End of Study, which is 96 h after dosing.

Pharmacokinetic samples will be analyzed after each cohort to examine the extent of drug exposure. Blood samples will be collected for determination of the active agent, its metabolites, and related substances at the following time-points: 0.25, 0.5, 1.0, 1.5, 2, 3, 4, 5, 6, 8, 10,12, 24, 30, 36, 48, 60, 72, 84 hr after dosing, and at End of Study. Up to 5 additional post-dosing blood sampling(s) may be performed and/or the timing of pharmacokinetic parameters draws may change if indicated after the analysis of blood samples from previous cohort(s). Samples will be collected after vital signs, pulse oximetry, and ECG are obtained.

Pharmacokinectic samples have been taken from the pharmaceutical composition according to the present invention comprising, 1 mg, 3, mg, 10 mg, 30 mg and 60 mg of 3α-hydroxy-3β-methoxymethyl-21-(1'-imidazolyl)-5α-pregnan-20-one. The mean plasma concentration of the subjects analyzed is provided in Table II.

**Table II**

| Time (h) | 1 mg composition | 3 mg composition | 10 mg composition | 30 mg composition | 60 mg composition |
|---|---|---|---|---|---|
| 0 | 0 | 0 | 0 | 0 | 0 |
| 0,25 | 0 | 0 | 0 | 0,26625 | 0,6355 |
| 0,5 | 0,02525 | 0,17275 | 2,447625 | 9,185 | 65,435 |
| 1 | 0,463 | 3,04025 | 20,22625 | 82,2 | 250,125 |
| 1,5 | 0,69675 | 3,8775 | 26,2125 | 111 | 267 |
| 2 | 0,97825 | 5,2975 | 25,375 | 106,1 | 251,5 |
| 3 | 1,1315 | 5,2625 | 21,9875 | 94,4 | 243,75 |
| 4 | 1,2125 | 4,7125 | 17,8 | 84,35 | 210,75 |
| 5 | 1,125 | 4,325 | 15,19875 | 70,375 | 176,25 |
| 6 | 1,12 | 3,58 | 13,5175 | 63,575 | 153 |
| 8 | 0,93075 | 2,925 | 10,2675 | 48,55 | 115,675 |
| 10 | 0,807 | 2,195 | 7,875 | 39 | 85,8 |
| 12 | 0,667 | 1,687 | 6,205 | 29,55 | 68,25 |
| 24 | 0,3755 | 0,90475 | 2,405 | 7,585 | 20,225 |
| 30 | 0,2835 | 0,53375 | 1,248625 | 9,445 | 12,625 |
| 36 | 0,2155 | 0,34175 | 1,21175 | 4,1225 | 7,25 |
| 48 | 0,119 | 0,155 | 0,685 | 2,145 | 3,2875 |
| 60 | 0 | 0,0545 | 0,3655 | 1,04125 | 1,4215 |
| 72 | 0 | 0,03225 | 0,198875 | 0,62375 | 0,7625 |
| 84 | 0 | 0 | 0,105 | 0,32025 | 0,417 |
| 96 | 0 | 0 | 0,022625 | 0,08175 | 0,21375 |

In addition, laboratory tests (hematology, serum chemistry, liver function tests, and urinalyses) will be done at 24 and 48 hours after dosing and at End of Study.

Pharmacodynamic assessment using SSS and CCT will be performed (with the same order as pre-dosing) at 0.5, 1, 1.5, 2, 3, 4, 5, 6, 8, 10, and 12 hr post dosing, after vital sign, pulse oximetry assessment, and pharmacokinetic sample collection. SSS will be performed prior to CCT.

During the study, the meals will be given at pretermined times and if necessary, the Investigator may decide to skip certain meal(s) for a subject depending on the degree of sedation at the time of such meal.

Subjects may leave the facility after the 48-hour procedures are completed, and return to the facility for subsequent procedures (vital sign measurements and blood draws) at specified times. Subjects may be asked to remain confined at the study site at the discretion of the Investigator due to any adverse events or other safety related reasons.

End-of-Study assessments will be performed at 96 hrs post-dosing or at the time of early discontinuation. This will include vital signs (blood pressures, pulse rate, respiratory rate and temperature), pulse oximetry, physical examination, ECG, laboratory tests (hematology, serum chemistry, liver function tests, and urinalysis) and pharmacokinectic blood sampling.

Having now fully described this invention, it will be understood by those of ordinary skill in the art that the same can be performed within a wide and equivalent range of conditions, formulations and other parameters without affecting the scope of the invention or any embodiment thereof All patents and publications cited herein are fully incorporated by reference herein in their entireties.

The following embodiments are also part of the description:
1. A pharmaceutical composition, comprising 3α-hydroxy-3β-methoxymethyl-21-(1'-imidazolyl)-5α-pregnan-20-one or a pharmaceutically-acceptable salt or solvate thereof, and one or more pharmaceutically-acceptable excipients, wherein the composition provides steady state plasma levels of 3α-hydroxy-3β-methoxymethyl-21-(1'-imidazolyl)-5α-pregnan-20-one in a range of from about 5 ng/mL to about 500 ng/mL.
2. The pharmaceutical composition according to claim item 1, wherein the composition provides steady state plasma levels of 3α-hydroxy-3β-methoxymethyl-21-(1'-imidazolyl)-5α-pregnan-20-one in a range of from about 50 ng/mL to about 500 ng/mL, from about 50 ng/mL to about 400 ng/mL, from about 50 ng/mL to about 325 ng/mL, from about 50 ng/mL to about 100 ng/mL or from about 100 ng/mL to about 250 ng/mL.
3. The pharmaceutical composition according to item 1, wherein the composition provides steady state plasma levels of 3α-hydroxy-3β-methoxymethyl-21-(1'-imidazolyl)-5α-pregnan-20-one in a range of from about 50 ng/mL to about 250 ng/mL.
4. The pharmaceutical composition according to item 3, wherein the pharmaceutical composition comprises from about 20 mg to about 40 mg, preferably about 30 mg, 3α-hydroxy-3β-methoxymethyl-21-(1'-imidazolyl)-5α-pregnan-20-one.
5. The pharmaceutical composition according to item 1, wherein the pharmaceutical composition is suitable for oral administration.
6. The pharmaceutical composition according to item 1, wherein the composition provides the steady state plasma levels of 3α-hydroxy-3β-methoxymethyl-21-(1'-imidazolyl)-5α-pregnan-20-one for a duration of from about 12 h to about 24 h following administration.
7. The pharmaceutical composition according to item 3, wherein the pharmaceutical composition is in the form of a hydrophilic matrix tablet, an encapsulated melt-extruded multiparticulates, a hydrophobic matrix tablet, melt-extruded granulation or controlled beads.
8. A method of treating a patient in need of treatment for generalized anxiety disorder, panic disorder, obsessive-compulsive disorder, post-traumatic stress disorder, social anxiety disorder, depression, alcohol addiction and/or dependence, premenstrual tension, premenstrual syndrome or premenstrual dysphoric disorder, comprising administering to said patient the pharmaceutical composition of any of items 1-7.
9. A use of a pharmaceutical composition according to any one of items 1-7 in the preparation of a medicament for the treatment of generalized anxiety disorder, panic disorder, obsessive-compulsive disorder, post-traumatic stress disorder, social anxiety disorder, depression, alcohol addiction and/or dependence, premenstrual tension, premenstrual syndrome or premenstrual dysphoric disorder.

## Claims

1. A pharmaceutical composition, comprising 3α-hydroxy-3β-methoxymethyl-21-(1'-imidazolyl)-5α-pregnan-20-one or a pharmaceutically-acceptable salt or solvate thereof, and one or more pharmaceutically-acceptable excipients, wherein the composition provides steady state plasma levels of 3α-hydroxy-3β-methoxymethyl-21-(1'-imidazolyl)-5α-pregnan-20-one in a range of from 5 ng/mL to 500 ng/mL for a duration of from 6 h to 24 h following administration.

2. The pharmaceutical composition according to claim 1, wherein the composition provides steady state plasma levels of 3α-hydroxy-3β-methoxymethyl-21-(1'-imidazolyl)-5α-pregnan-20-one in a range of from 5 ng/mL to 500 ng/mL for a duration of from 12 h to 24 h following administration.

3. The pharmaceutical composition according to claim 1 or 2, wherein the composition provides steady state plasma levels of 3α-hydroxy-3β-methoxymethyl-21-(1'-imidazolyl)-5α-pregnan-20-one in a range of from 50 ng/mL to 500 ng/mL, from 50 ng/mL to 400 ng/mL, from 50 ng/mL to 325 ng/mL, from 50 ng/mL to 100 ng/mL or from 100 ng/mL to 250 ng/mL.

4. The pharmaceutical composition according to claim 1 or 2, wherein the composition provides steady state plasma levels of 3α-hydroxy-3β-methoxymethyl-21-(1'-imidazolyl)-5α-pregnan-20-one in a range of from 50 ng/mL to 250 ng/mL.

5. The pharmaceutical composition according to any one of claims 1 to 4, wherein the pharmaceutical composition comprises from 20 mg to 40 mg, preferably 30 mg, 3α-hydroxy-3β-methoxymethyl-21-(1'-imidazolyl)-5α-pregnan-20-one.

6. The pharmaceutical composition according to any one of claims 1 to 5, wherein the pharmaceutical composition is suitable for oral administration.

7. The pharmaceutical composition according to claim 4, wherein the pharmaceutical composition is in the form of a hydrophilic matrix tablet, an encapsulated melt-extruded multiparticulate, a hydrophobic matrix tablet, melt-extruded granulation or controlled release beads.

8. The pharmaceutical composition according to any one of claims 1 to 7 for use in the treatment of anxiety.

9. The pharmaceutical composition according to claim 8, for use in the treatment of a generalized anxiety disorder, panic disorder, obsessive compulsive disorder, post-traumatic stress disorder or social anxiety disorder.
